# EUROPEAN PATENT APPLICATION

(11) **EP 4 678 195 A1**
(43) Date of publication of application: **14.01.2026**
(21) Application number: 24766467.5
(22) Date of filing: 06.03.2024
(51) Int. Cl.: A61L 12/08, A61L 12/02, A61L 12/10, G02C 13/00

(54) **ELECTROCHEMICAL STERILIZATION AND PROTEIN REMOVAL METHOD AND DEVICE**

(30) Priority: 07.03.2023 CN 202310211856
(71) Applicant: Suzhou 3N Biological Technology Co., Ltd, Suzhou, Jiangsu 215000 (CN)
(72) Inventor: SUN, Bixia, Suzhou, Jiangsu 215000 (CN)
(74) Representative: Gille Hrabal Partnerschaftsgesellschaft mbB Patentanwälte
(86) International application number: PCT/CN2024/080290
(87) International publication number: WO 2024/183752

(57) **Abstract**

Disclosed in the present patent are an electrochemical sterilization and protein removal method and device. The method comprises: pouring a sodium chloride solution into care compartments, the care compartments being each internally provided with at least a first electrode and a second electrode, and the pH value range of the sodium chloride solution being 8-12; putting contact lenses in the sodium chloride solution in the care compartments; conducting the first and second electrodes to a power supply, one of the first and second electrodes serving as anodes, and the other one being cathodes; the anodes electrolyzing Cl-in the sodium chloride solution into Cl2, and Cl2 being dissolved in the solution to generate HCL and HCLO; and controlling the pH value of the solution in the care compartments to be 8-12, and controlling the mass concentration of CIO- in the solution to be 0.1-0.4%, wherein the CIO- can sterilize the contact lenses and degrade denatured proteins on same, and under the effect of an electric field force, the degraded denatured proteins move in a direction towards electrodes the polarity of which is opposite to the charges carried by the denatured proteins themselves. The present invention has excellent sterilization and protein removal effects, effectively degrades proteins, achieves instant sterilization, and can reach the sterile grade just within 10 minutes. The present invention does not cause damage to contact lenses, has excellent stability, and requires short time for sterilization and protein removal.

## Description

### BACKGROUND OF THE INVENTION

### 1. Technical Field

The present invention relates to the field of contact lens sterilization and protein removal, and particularly to an electrochemical sterilization and protein removal method and device.

### 2. Description of Related Art

Currently, cleaning and care for contact lenses, such as sterilization and protein removal, commonly employ manual rubbing methods in the market. This approach generally requires 2-4 hours or more, is time-consuming and labor-intensive, and manual rubbing can lead to a series of issues, such as insufficient rubbing force resulting in inadequate removal of deposited proteins, excessive rubbing force causing lens breakage, uneven rubbing force leading to lens deformation or scratches, inadequate finger cleaning causing lens damage or bacterial infection, and finger contact with care solutions potentially harming the skin, etc.

Current care solutions or conventional sterilization and protein removal devices lack specific protective measures, which can easily cause contact lenses to fade or partially fade, making it difficult to distinguish between left and right eyes or affecting aesthetic appearance, thereby inconveniencing users and impairing normal use of contact lenses. The applicant's previously filed technical patents related to contact lens care, such as patent application numbers: 202011505217X, 2020107601353, and 2020116347313, have addressed some of the care issues for contact lenses to a certain extent, but still exhibit certain shortcomings. Moreover, there remains significant room for improvement in the efficiency and effectiveness of contact lens cleaning.

Care solutions can also easily corrode contact lens coatings, not only affecting the lifespan of the lenses but also potentially causing eye damage if the corroded coating enters the eye. Additionally, if care solutions are used to clean contact lenses and the lenses are worn directly without rinsing, it can cause harm to the eyes.

### BRIEF SUMMARY OF THE INVENTION

To address at least one of the technical problems existing in the prior art, the present invention provides an electrochemical sterilization and protein removal method and device. By utilizing electrochemical technology to generate certain oxidative substances such as hypochlorite from sodium chloride solution, the invention achieves excellent sterilization and protein removal effects, effectively degrading proteins and providing instant sterilization, reaching sterilization levels within 10 minutes. Proper control of relevant parameters ensures that the sterilization and protein removal process does not damage the contact lenses themselves, offering good stability and low toxicity. The use of an electrochemical sterilization and protein removal device for automatic cleaning eliminates the need for manual rubbing, avoids contact with care solutions, and reduces harm to the human body. Short sterilization and protein removal time.

In one aspect, the present invention provides an electrochemical sterilization and protein removal method, specifically comprising the following steps:

S1: Pour a sodium chloride solution into a care chamber, wherein the care chamber is provided with at least a first electrode and a second electrode, both the first electrode and the second electrode being chlorine evolution electrodes, the pH range of the sodium chloride solution is 8-12, and a contact lens is placed in the sodium chloride solution within the care chamber;

S2: Connect the first electrode and the second electrode to a power supply, wherein one of the first electrode and the second electrode serves as an anode and the other as a cathode, an oxidation reaction occurs at the anode end, and a reduction reaction occurs at the cathode end, the cathode electrolyzes H₂O in the sodium chloride solution to produce H₂ and OH⁻; the anode electrolyzes Cl⁻ in the sodium chloride solution to produce Cl₂, and Cl₂ dissolves in the solution to generate HCl and HClO;

S3: Control the pH of the solution in the care chamber to be within the range of 8-12, control the mass concentration of ClO⁻ in the solution to be 0.1%-0.4%, the ClO⁻ can sterilize the contact lens and degrade denatured proteins and some non-denatured proteins on the contact lens, an electric field force is formed between the cathode and the anode, the degraded denatured proteins and some non-denatured proteins move toward the electrode with opposite charge under the action of the electric field force, and the non-degraded non-denatured proteins also move toward the electrode with opposite charge under the action of the electric field force, achieving sterilization and protein removal of the contact lens.

As a preferred embodiment of the electrochemical sterilization and protein removal method described in this patent, the NaCl solution is a physiological saline with a mass concentration of 0.9%, the turn-on voltage of the power supply is not less than 1.1V, and the stable voltage range after power supply connection is 3.5-6.5V;

In step S3, after the power supply is connected and the reaction proceeds for 10-60 minutes, the power supply is disconnected, and the contact lens care is completed.

As a preferred embodiment of the electrochemical sterilization and protein removal method described in this patent, the bottom heights of the first electrode and the second electrode are both higher than the bottom height of the care chamber, the contact lens is placed horizontally in the NaCl solution within the care chamber, the top height of the contact lens is lower than the bottom height of the probe, and the distance from the top of the contact lens to the liquid surface of the NaCl solution is not less than 4mm; or

The contact lens is placed vertically in the NaCl solution within the care chamber, the distance from the contact lens to the anode is not less than 4mm, and the distance from the contact lens to the liquid surface of the NaCl solution is not less than 4mm.

As a preferred embodiment of the electrochemical sterilization and protein removal method described in this patent, in steps S2 and S3, after the first electrode and the second electrode are connected to the power supply, the first electrode and the second electrode switch between the cathode and the anode at certain time intervals.

As a preferred embodiment of the electrochemical sterilization and protein removal method described in this patent, the first electrode and the second electrode are switched between the cathode and the anode every 0.5-2 hours.

As a preferred embodiment of the electrochemical sterilization and protein removal method described in this patent, the NaCl solution further includes alkaline substances, which comprise one or more of NaOH, Na₂CO₃, and NaHCO₃, to adjust the pH range of the sodium chloride solution in step S1 to 8-12.

As a preferred embodiment of the electrochemical sterilization and protein removal method described in this patent, it further includes step S4;

S4: After the completion of step S3 care, rinse the contact lens with sterile physiological saline or a multi-purpose eye care solution. After rinsing, apply lubricating drops to the concave surface of the contact lens and wear the contact lens.

On the other hand, this patent provides an electrochemical sterilization and protein removal device, comprising a care chamber and a base;

The care chamber includes a accommodation slot, a sunken slot , a first electrode, and a second electrode; the sunken slot is connected to the accommodation slot, the sunken slot is located below the accommodation slot, the first electrode and the second electrode are both arranged in the accommodation slot, and the base is capable of supplying electrical power to the first electrode and the second electrode;

When the first electrode and the second electrode are energized, the electrical properties of the first electrode and the second electrode are opposite, one being the cathode and the other being the anode. Under the working condition of adding a sodium chloride solution with the pH greater than 8 into the care chamber, oxidation occurs at the anode and reduction occurs at the cathode, the cathode electrolyzes the sodium chloride solution to generate H₂ and OH⁻; the anode electrolyzes Cl⁻ in the sodium chloride solution intoCl₂, Cl₂ dissolves in the solution to produce HCl and HClO; the ClO⁻ can sterilize the contact lens and degrade denatured proteins and some non-denatured proteins on the contact lens. The degraded denatured proteins and some non-denatured proteins move toward the electrode with the opposite charge under the electric field force formed between the anode and the cathode, and the non-degraded non-denatured proteins also move toward the electrode with the opposite charge under the electric field force formed between the anode and the cathode, to achieve sterilization and protein removal of the contact lens.

As a preferred embodiment of the electrochemical sterilization and protein removal device described in this patent, the first electrode and the second electrode are both chlorine-evolving electrodes, and under working conditions, the mass concentration of ClO⁻in the solution within the care chamber is 0.1%-0.4%.

As a preferred embodiment of the electrochemical sterilization and protein removal device described in this patent, both the first electrode and the second electrode are chlorine evolution electrodes;

The NaCl solution is a physiological saline with a mass concentration of 0.9%, the starting voltage of the first electrode and the second electrode when connected to the power supply is not less than 1.1V, the stable voltage range after power connection is 3.5-6.5V, and the operating time after power connection is 10-60 minutes.

As a preferred embodiment of the electrochemical sterilization and protein removal device described in this patent, it further includes a horizontal lens clamp, which can limit the contact lens to be placed horizontally in the sinking slot to prevent the contact lens from being too close to the electrodes and causing oxidative discoloration, and the distance from the top of the contact lens to the surface of the sodium chloride solution is not less than 4mm.

As a preferred embodiment of the electrochemical sterilization and protein removal device described in this patent, it further includes a vertical lens clamp, which can limit the contact lens to be placed vertically in the care chamber, the entire contact lens is arranged at a vertical angle to the first electrode and the second electrode, the distance between the contact lens and the anode in the care chamber is not less than 4mm, and the distance from the top of the contact lens to the surface of the sodium chloride solution is not less than 4mm.

As a preferred embodiment of the electrochemical sterilization and protein removal device described in this patent, the first electrode and the second electrode are symmetrically arranged in the accommodating slot, and both the first electrode and the second electrode are close to the side wall of the accommodating slot.

As a preferred embodiment of the electrochemical sterilization and protein removal device described in this patent, the horizontal lens clamp includes a mounting base, a support member, and a limiting member, the support member is vertically arranged on the mounting base, the limiting member is provided with a limiting hole, during use, the length direction of the limiting hole is consistent with the horizontal direction, there are at least two limiting members, and the contact lens is limited in the limiting hole.

As a preferred embodiment of the electrochemical sterilization and protein removal device described in this patent, the vertical lens clamp includes a fixed base and at least two accommodating members, the accommodating members are vertically arranged on the fixed base, the accommodating members are provided with accommodating holes, during use, the length direction of the accommodating hole is consistent with the vertical direction, there are at least two accommodating members, and the contact lens is limited in the accommodating hole.

As a preferred embodiment of the electrochemical sterilization and protein removal device described in this patent, it further includes a control system, the control system is provided with an electrode switching module, and the electrode switching module can switch the first electrode and the second electrode between the cathode and the anode through circuit control.

Compared with the prior art, the technical solution described in this patent has at least one or more of the following beneficial effects:

The present invention is an application technology that electrochemically generates certain oxidative substances such as hypochlorite from sodium chloride solution, which has excellent sterilization and protein removal effects, effectively degrades proteins, and achieves sterilization level in just 10 minutes. By appropriately controlling relevant parameters, the sterilization and protein removal process does not damage the contact lens itself. If not controlled properly, it may also cause damage to the contact lens itself, such as discoloration of the contact lens. Excellent stability and low toxicity/side effects. Automatic cleaning using electrochemical sterilization and protein removal device, eliminating manual scrubbing and contact with care solution, reducing harm to human body. Short sterilization and protein removal time for contact lenses, requiring only 10-60 minutes. Conventional care solutions take at least 2-4 hours and require manual scrubbing, being time-consuming and labor-intensive.

At least the anode is a chlorine evolution electrode. In sodium chloride solution, oxidation occurs at the anode to release chlorine gas from chloride ions in the solution. The chlorine evolution electrode exhibits strong corrosion resistance, excellent chlorine/acid resistance, good stability, oxidation resistance, long service life, preventing anode reactions or byproduct generation that could contaminate electrolyte or cathode products; facilitates bubble discharge between/on electrode surfaces, effectively reducing voltage in electrolytic cell; easy to shape with high precision. Common metal electrodes mainly include ruthenium, iridium, and platinum series. Other inert electrodes (e.g., graphite, graphene materials) can also serve as chlorine evolution electrodes provided material morphology stability is maintained. The electrode may also be a coated electrode, preferably using titanium or titanium alloy substrate coated with ruthenium, iridium, platinum series metal coatings. The cathode and anode are preferably symmetrically distributed on both sides of the care chamber, positioned close to sidewalls for better protein removal.

Commercially available care solutions tend to damage contact lenses with sterilization rates typically around 90%. The present invention achieves instant sterilization, not only with excellent sterilization effect but also at a high sterilization speed, achieving a 99% bacterial kill rate and 99% fungal kill rate within 3 minutes. Adjusting relevant parameters can further increase the sterilization rate for bacteria and fungi, achieving a sterilization level by killing Bacillus subtilis within 10 minutes. Moreover, commercially available care solutions cannot be directly applied to the eyes. If users forget to rinse after completing contact lens care and wear the lenses directly, residual care solution on the lenses may cause eye damage. If users forget to rinse and wear the contact lenses after cleaning with the present invention, no eye damage will occur. Additionally, AB solution contains potassium bromide, which generates liquid bromine during use. Liquid bromine can corrode the coating of contact lenses, causing coating detachment. Detached coating not only affects the lifespan of the lenses but may also lead to inflammation if it enters the eyes. Furthermore, residual bromine on the lenses may compromise safety.

After the first electrode and second electrode of this patent are energized, the sodium chloride solution generates various oxidative substances and free radicals, particularly hypochlorous acid and hypochlorite ions, which can disrupt protein peptide chains. The preferred mass concentration of hypochlorite ions in this patent is 0.1%. The concentration of hypochlorite ions in Solution A of AB solution is 0.375%. Although AB solution achieves good sterilization and protein removal effects, contact lenses should not be soaked in AB solution for extended periods, as it may cause damage such as discoloration and corrosion. Due to the incorporation of electrophoresis technology, the preferred 0.1% concentration of hypochlorite ions in this patent is less than one-third of that in AB solution, yet achieves excellent sterilization and protein removal effects. It is safer and more convenient to use, and by appropriately controlling relevant parameters, it can reduce lens discoloration and avoid coating damage and corrosion.

The mass concentration range of hypochlorite ions in this patent is controlled between 0.1%-0.4%. If the concentration range is 0.01%-0.1%, although solutions containing hypochlorite ions in this range can be directly applied to the eyes and mucous membranes with good sterilization effects, their protein removal effect is weaker. When the concentration range of hypochlorite ions is 0.4%-1%, sterilization and protein removal effects are better. However, high concentrations of hypochlorite ions remain stable only in strongly alkaline environments and may damage non-antioxidant materials in principle. Therefore, overall, a hypochlorite ion concentration of 0.1%-0.4% is the preferred choice.

Through extensive long-term research, investigators have identified the preferred concentration range for hypochlorite ions and determined experimental conditions that maintain the concentration within the optimal range. These include an optimal sodium chloride solution mass concentration of 0.9%, a minimum activation voltage of 1.1V for the first and second electrodes, a stable voltage range of 3.5-6.5V after power is applied, and when the contact lens is placed horizontally in the care chamber, the top height of the lens should be lower than the bottom height of the probe, with a distance of at least 4mm from the NaCl solution surface. Alternatively, when the lens is placed vertically, it should be at least 4mm from the anode and 4mm from the NaCl solution surface, with a reaction time of 10-60 minutes after power is applied.

The sodium chloride solution of this patent can generate hydroxyl radicals (OH•), oxygen radicals (O•), chlorine radicals (Cl•), hydrogen peroxide (H₂O₂), ozone (O₃), hypochlorite ions (ClO⁻), hypochlorous acid (HClO), chlorine gas (Cl₂), hydrogen ions (H⁺), and other substances under the action of an electric field. Combined with electrophoresis under specific conditions, it achieves excellent protein removal and sterilization effects, comparable to AB solution. Moreover, compared to AB solution, the concentration of hypochlorite ions generated in the reaction is less than one-third of that in AB solution, making it safer to use and more convenient to operate.

After the first electrode and the second electrode of this patent are connected to a power source, one of them acts as the anode and the other as the cathode. Oxidation occurs at the anode, while reduction occurs at the cathode. The cathode electrolyzes H₂O in the sodium chloride solution to produce H₂ and OH⁻; the anode electrolyzes Cl⁻ in the sodium chloride solution to produce Cl₂, which dissolves in the solution to form HCl and HClO. The ClO⁻ can sterilize contact lenses and degrade denatured proteins and some undenatured proteins on the lenses. An electric field force forms between the cathode and anode, causing the degraded denatured proteins and some undenatured proteins to move toward the electrode with the opposite charge under the electric field force. Undegraded undenatured proteins also move toward the electrode with the opposite charge under the electric field force, achieving sterilization and protein removal for contact lenses. In an alkaline environment, HClO decreases, preventing lens discoloration caused by HClO. Most hypochlorite ions exist as NaClO, which has milder sterilization and protein removal effects without bleaching properties, thus avoiding lens discoloration. The ClO⁻ ions in NaClO can sterilize and degrade proteins, and, with the assistance of electrophoresis, achieve protein removal.

This invention includes an electrode switching module. After the first electrode and the second electrode are connected to a power source, the electrode switching module controls the circuit to switch the first and second electrodes between the cathode and anode at certain time intervals. This prevents the accumulation of single or similar ions near the cathode or anode, ensuring uniform concentration and avoiding adverse effects such as lens discoloration caused by HClO accumulation. Specifically, after the first electrode and the second electrode are connected to a power source, H₂O in the sodium chloride solution at the cathode is electrolyzed to produce H₂ and OH⁻, with the reaction e + 2H₂O = H₂ and OH⁻. OH⁻ accumulates, resulting in a pH greater than 7 in the solution near the cathode. Near the anode, Cl⁻ in the sodium chloride solution is electrolyzed to produce Cl₂, which dissolves in water to form HCl and HClO. The accumulation of HCl and HClO results in a pH less than 7 in the solution near the anode. Switching the first electrode and the second electrode between the cathode and anode can neutralize HCl. The purpose is to facilitate greater neutralization into NaClO, reducing the impact on contact lens color. Meanwhile, the switching electrode can uniformly mix other ions or molecules generated at the cathode and anode ends, facilitating uniform sterilization and protein removal for contact lenses.

Compared to the traditional manual rubbing method for cleaning contact lenses on the market, the electrophoretic dissociation's electric field force in this patented sterilization and protein removal technology acts specifically on charged particles on contact lenses, such as proteins, bacteria, and fungi, more effectively separating denatured proteins from the lenses without damaging the lenses themselves. This avoids issues such as insufficient rubbing force leading to inadequate removal of deposited proteins, excessive rubbing force causing lens breakage, uneven rubbing force resulting in lens deformation or scratches, and insufficient finger cleaning causing lens scratches or bacterial infections.

The electrochemical sterilization and protein removal technology of the present invention can kill microorganisms such as bacteria, and its sterilization principle mainly includes the following aspects:

### 1. Electrolysis produces hypochlorous acid, blocking the bacterial protein synthesis pathway

Electrolysis of sodium chloride solution produces hypochlorous acid, which is a strong oxidizing agent. The sterilization principle of hypochlorous acid is to oxidize and denature proteins in microorganisms, preventing them from normal replication and thus losing viability. Hypochlorous acid is 80 times more effective than hypochlorite ions in killing microorganisms and can generate hydroxyl radicals that act on different bacteria. Moreover, long-term use does not lead to bacterial resistance to electrolyzed physiological saline, and it has no toxic side effects on the cornea.

2. Electroincorporation: Under the influence of an electric field, substances are incorporated into cells from the solution, known as electroincorporation. Based on the effects of an external electric field on bacterial growth, activity, metabolism, morphology, and movement, combined with hydroxyl radicals (OH •), oxygen radicals (O•), chlorine radicals (Cl•), hydrogen peroxide (H₂O₂), ozone (O₃), hypochlorite ions (ClO⁻), hypochlorous acid (HClO), chlorine gas (Cl₂), and hydrogen ions (H⁺) generated by electrolysis of chloride-containing solutions, a new sterilization and protein removal technology has been developed. Applying an appropriate current can, on one hand, interfere with gene expression in cells, inhibit ATPase activity, reduce protein content within bacterial cells, affect intracellular free radical reactions and the synthesis of biological macromolecules, and suppress cell proliferation by neutralizing the negative charge on cell surfaces, ultimately leading to cell apoptosis, senescence, and death. On the other hand, it can enhance the permeability of microbial cells, causing electroporation, allowing hydroxyl radicals (OH•), oxygen radicals (O•), chlorine radicals (Cl•), hydrogen peroxide (H₂O₂), ozone (O₃), hypochlorite (ClO⁻), hypochlorous acid (HClO), chlorine gas (Cl₂), hydrogen ions (H⁺), etc., to penetrate microbial cells, generating cytotoxicity, inducing structural and functional disorders in cells, thereby achieving inactivation, sterilization, and disinfection effects.

### 3. Micro-electrolysis generates active substances that disrupt the chain structure of cellular organic matter

Under the influence of a physical field, the applied current density is controlled, and the excited electrons transfer from the anode to the cathode through the medium of water. During this transfer process, oxidizing substances such as free radicals, ClO⁻, Cl⁻, OH⁻, H₂O₂, etc., are generated. These active substances can react rapidly with any molecules in living cells, such as sugars, phospholipids, and organic acids, damaging the cell membrane and penetrating into the cell to disrupt the chain structure of organic matter. They oxidize bacterial cell RNA and DNA, leading to inactivation or death. Additionally, bacteria in water generally carry a negative charge and migrate toward the anode, where they accumulate and may discharge directly, causing death.

### 4. Microcurrent punctures bacterial cell walls, rapidly oxidizing bacterial RNA\DNA

The direct action of current on cell walls can mechanically damage bacteria in the solution, enhancing the oxidation of bacterial and viral RNA\DNA.

### 5. Microcurrent breaks bacterial clusters, reducing bacterial tolerance

The action of current alters the relative dispersion environment of bacteria in water, significantly reducing the stability of aggregated mixtures. When bacteria and viruses are considered as a colloidal system composed of water, proteins, and nucleic acids, microcurrent reduces the stability of bacteria, thereby lowering their tolerance to hypochlorous acid.

### 6. Electron-activated water enhances the contact surface between hypochlorous acid and bacteria

The properties of water depend on the structural changes of water molecule electrons, primarily the distribution, shape, and orientation changes of electron clouds. Studies have confirmed that electron clouds can be altered by external environments. Under low voltage and microcurrent conditions, the four pairs of electrons in water molecules transition from lower to higher orbitals, elevating the electron energy levels. This results in the loss of potential energy of activated water molecules, causing a decrease in their potential. The potential difference between water molecules and interfaces (microbial surfaces) is reduced. Such changes may affect the aggregation state of bacteria and viral particles. Moreover, common hydrophilic bacteria on contact lenses, such as Streptococcus viridans, Pseudomonas aeruginosa, and Staphylococcus, are more likely to come into contact with hypochlorous acid after microcurrent treatment, thereby enhancing bactericidal efficiency.

Therefore, the electrophoretic dissociation protein removal and sterilization method described in the present invention achieves multiple effects of protein removal and sterilization.

Additional aspects and advantages of the invention will be set forth in part in the description that follows, and in part will be obvious from the description, or may be learned by practice of the invention.

### BRIEF DESCRIPTION OF THE SEVERAL VIEWS OF THE DRAWINGS

To describe the technical solutions of the present invention more clearly, the following briefly introduces the accompanying drawings required for describing the embodiments or the prior art. Obviously, the accompanying drawings in the following description show merely some embodiments of the present invention, and a person of ordinary skill in the art may still derive other drawings from these accompanying drawings without creative efforts.
FIG. 1 is an exploded schematic structural view of the electrochemical sterilization and protein removal device according to the present patent;
FIG. 2 is an exploded schematic structural view of the electrochemical sterilization and protein removal device according to the present patent;
FIG. 3 is a three-dimensional schematic structural view of the care chamber according to the present patent;
FIG. 4 is a cross-sectional three-dimensional schematic structural view of the care chamber according to the present patent;
FIG. 5 is a three-dimensional schematic structural view of the lateral lens clamp and cover body according to the present patent;
FIG. 6 is an exploded schematic view of the lateral lens clip and cover assembly according to the present patent;
FIG. 7 is a perspective schematic view of the vertical lens clip and cover assembly according to the present patent;
FIG. 8 is an exploded schematic view of the vertical lens clip and cover assembly according to the present patent;
FIG. 9 is a circuit schematic diagram of the electrochemical sterilization and protein removal device according to the present patent;
FIG. 10 is an SEM image of a contact lens not maintained using the method of the present patent;
FIG. 11 is an SEM image of a contact lens after six months of maintenance with AB solution;
FIG. 12 is another SEM image of a contact lens after six months of maintenance with AB solution;
FIG. 13 is an SEM image after 300 cleaning cycles using the electrochemical sterilization and protein removal device;
FIG. 14 is a graph showing the effect of initial physiological saline pH and stabilized voltage on ClO⁻ concentration in the electrochemical sterilization and protein removal process according to the present patent.

Wherein, 1-maintenance chamber, 2-base, 3-lens clip, 5-contact lens, 11-accommodation slot, 12-sunken slot, 13-first electrode, 14-second electrode, 16-cover assembly, 31-lateral lens clip, 311-mounting base, 312-support member, 313-limit member, 314-limit hole, 32-vertical lens clip, 321-fixed base, 322-accommodation member, 323-accommodation hole.

### DETAILED DESCRIPTION OF THE INVENTION

Embodiments of the present invention are described in detail below, examples of which are illustrated in the accompanying drawings, wherein like or similar reference numerals designate like or similar elements or elements having like or similar functions throughout. The embodiments described below with reference to the accompanying drawings are exemplary and are intended to explain the present invention, but should not be construed as limiting the present invention. Based on the embodiments of the present invention, all other embodiments obtained by those of ordinary skill in the art without creative efforts shall fall within the protection scope of the present invention.

In the description of the present invention, it should be understood that the orientation or positional relationship indicated by the terms "upper," "lower," "top," "bottom," "inner," "outer," "front end," "rear end," "both ends," "one end," "the other end," etc., is based on the orientation or positional relationship shown in the accompanying drawings, and is merely for the convenience of describing the present invention and simplifying the description, rather than indicating or implying that the referred device or element must have a specific orientation, be constructed and operated in a specific orientation, and thus should not be construed as limiting the present invention. In the description of the present invention, the meaning of "a plurality" is two or more, unless otherwise explicitly and specifically defined. Furthermore, the terms "first," "second," "third," "fourth," "fifth" are used for descriptive purposes only and should not be construed as indicating or implying relative importance.

In the description of the present invention, unless otherwise explicitly specified and defined, the terms "provided with," "equipped with," "connected," "mounted with," "sleeved," "opened," "fixed," etc., should be understood broadly. For example, the connection may be a fixed connection, a detachable connection, or an integral connection; it may be a mechanical connection or an electrical connection; it may be a direct connection or an indirect connection through an intermediate medium, and it may be the internal communication of two elements or the interaction relationship between two elements. For those of ordinary skill in the art, the specific meanings of the above terms in the present invention can be understood according to specific circumstances.

In one aspect, the present invention provides an electrochemical sterilization and protein removal method, the specific steps of which are as follows:
S1: Pour sodium chloride solution into the care chamber, wherein the care chamber is provided with at least a first electrode and a second electrode, the pH value of the sodium chloride solution is greater than 8-12, and the contact lens is placed in the sodium chloride solution within the care chamber; of course, halide solutions such as potassium chloride may also be used;
S2: Connect the first electrode and the second electrode to a power source, wherein one of the first electrode and the second electrode serves as an anode and the other as a cathode, oxidation occurs at the anode end and reduction occurs at the cathode end, the cathode electrolyzes H₂O in the sodium chloride solution to produce H₂ and OH⁻; the anode electrolyzes Cl⁻ in the sodium chloride solution into Cl₂, and Cl₂ dissolves in the solution to produce HCl and HClO;
S3: Control the pH value of the solution in the care chamber to be 8-12, control the mass concentration of ClO⁻ in the solution to be 0.1%-0.4%, the ClO⁻ can sterilize the contact lens and degrade denatured proteins and some undenatured proteins on the contact lens, an electric field force is formed between the cathode and the anode, the degraded denatured proteins and some undenatured proteins move towards the electrode with the opposite charge under the action of the electric field force, the undenatured proteins that are not degraded also move towards the electrode with the opposite charge under the action of the electric field force, achieving sterilization and protein removal of the contact lens. Wherein, the contact lens includes corneal contact lenses, OK lenses, and scleral lenses, etc.

In a preferred embodiment, the NaCl solution is physiological saline with a mass concentration of 0.9%, that is, the concentration of the NaCl solution is 0.009g/ml, the starting voltage of the power source is not less than 1.1V, the stable voltage range after power connection is 3.5-6.5V; in step S3, after the power is connected and reacts for 10-60 minutes, the contact lens care process is completed. If the starting voltage is too low, the cathode and anode cannot promote the redox reaction of ions in the sodium chloride solution.

In a preferred embodiment, the contact lens can be placed horizontally or vertically in the NaCl solution within the care chamber. In one case, the contact lens is placed horizontally in the NaCl solution within the care chamber, the bottom heights of the first electrode and the second electrode are both higher than the bottom height of the care chamber, the top height of the contact lens is lower than the bottom height of the probe, and the distance from the top of the contact lens to the liquid surface of the NaCl solution is not less than 4mm. In one case, the contact lens is placed vertically in the NaCl solution within the care chamber, the distance from the contact lens to the anode is not less than 4mm, and the distance from the contact lens to the liquid surface of the NaCl solution is not less than 4mm. If electrode switching is performed on the first electrode and the second electrode, the contact lens is preferably set at the middle position between the first electrode and the second electrode, with distances to both the first electrode and the second electrode not less than 4mm. If the first electrode and the second electrode do not undergo electrode switching, the distance between the contact lens and the anode shall be no less than 4 mm. Of course, the contact lens may also be positioned at the midpoint between the first electrode and the second electrode.

In a preferred embodiment, in steps S2 and S3, after the first electrode and the second electrode are connected to the power supply, they switch between the cathode and the anode at certain time intervals. Preferably, the first electrode and the second electrode switch between the cathode and the anode every 0.5 to 2 minutes. More preferably, the first electrode and the second electrode switch between the cathode and the anode every 1 minute.

In a preferred embodiment, the alkaline chlorine-containing solution is a mixture of NaCl and an alkaline substance, wherein the sodium chloride solution includes the alkaline substance, and the alkaline substance comprises one or more of NaOH, Na₂CO₃, and NaHCO₃. Even more preferably, the alkaline substance is NaOH.

In the example, both the first electrode and the second electrode are chlorine-evolving electrodes. In the sodium chloride solution, an oxidation reaction occurs at the anode terminal, releasing chlorine gas from the chloride ions in the solution. The chlorine evolution electrode exhibits strong corrosion resistance, excellent chlorine/acid resistance, good stability, oxidation resistance, long service life, preventing anode reactions or byproduct generation that could contaminate electrolyte or cathode products; facilitates bubble discharge between/on electrode surfaces, effectively reducing voltage in electrolytic cell; easy to shape with high precision. Common metal electrodes mainly include ruthenium, iridium, and platinum series. Other inert electrodes (e.g., graphite, graphene materials) can also serve as chlorine evolution electrodes provided material morphology stability is maintained. The electrode substrate preferably adopts a titanium alloy substrate or a platinum-plated substrate. The cathode and anode are preferably symmetrically distributed on both sides of the care chamber, positioned close to sidewalls for better protein removal. In the example, both the first electrode and the second electrode are probes; of course, the first electrode and the second electrode can also be electrode sheets, which can be adjusted as needed.

In a preferred embodiment, it further includes step S4;
S4: After the completion of step S3 care, rinse the contact lens with sterile physiological saline or a multi-purpose eye care solution. Of course, after rinsing, it is preferable to drop lubricant into the concave surface of the contact lens before wearing it.

### Example

Based on the influence of the aforementioned parameters, the inventors conducted a series of experiments to verify the impact of each parameter on the care results of contact lenses, such as sterilization and protein removal. This experiment used the CRT contact lens with the lightest pigment currently available on the market for testing, employing a 0.9% mass concentration of physiological saline. The initial pH value of the physiological saline was adjusted, along with the care chamber's starting voltage, stable voltage, placement method of the contact lens, distance from the liquid surface, distance from the anode, whether to switch electrodes, switching frequency, and working time. The final pH value and the final concentration of ClO⁻ at the end of the care process were measured, ultimately detecting the protein removal rate, sterilization rate including bacterial and fungal sterilization rates, and the impact of sterilization and protein removal care on the color of the contact lens. The experimental data and results are shown in Table 1 below:

**Table 1 Data and results of cleaning care such as sterilization and protein removal for contact lenses using the device**

| Init ial | Fin al | Star ting | Sta ble | Plac eme | Dista nce | Distan ce | Elect rode | Swit chin | Wor king | Final conc | Prote in | Steri lizati | Effect on |
|---|---|---|---|---|---|---|---|---|---|---|---|---|---|
| | | | | Hori zont al place ment | 1-4 | 0-4 | Yes | 5s-1 20s | 30mi n | 0.13 5 % | 80% | Bact eria ≥99. 999 % Fung i ≥99. 999 % | No fading within 9 times |
| | | | | | | | No | | | | 85% | | No fading within 1-5 times |
| 8 | 8-9 | >1. 1 V | 5.0 V | | ≥4 | ≥4 | Yes | 5s-1 20s | 30mi n | | 70% | | No fading within 20 times |
| | | | | | | | No | | | | 75% | | No fading within 14 times |
| | | | | Verti cal place ment | 1-4 | 0-4 | Yes | 5s-1 20s | 30mi n | | 85% | | No fading within 1-3 times |
| | | | | | | | No | | | | 90% | | No fading within 2-3 times |
| | | | | | ≥4 | ≥4 | Yes | 5s-1 20s | 30mi n | | 80% | | No fading within 10 times |
| | | | | | | | No | | | | 85% | | No fading within 8 times |
| 9 | 9~1 0 | > 0.5 7 V | 5.0 V | Hori zont al place ment | 1-4 | 0-4 | Yes | 5s-1 20s | 30mi n | 0.14 2 % | 82% | | No fading within 20 times |
| | | | | | | | No | | | | 85% | | No fading within 16 times |
| | | | | | ≥4 | ≥4 | Yes | 5s-1 20s | 30nii n | | 73% | Bact eria ≥99. 999 % Fung i ≥99. 999 % | No fading within 45 times |
| | | | | | | | No | | | | 77% | | No fading within 38 times |
| | | | | Verti cal place ment | 1-4 | 0-4 | Yes | 5s-1 20s | 30 min | | 85% | | No fading within 6-8 times |
| | | | | | | | No | | | | 90% | | No fading within 4-6 times |
| | | | | | ≥4 | ≥4 | Yes | 5s-12 | 30mi | | 82% | | No |
| | | | | | | | No | | | | 85% | | No |
| 10 | 1.0 11 | > 0.5 V | 5.0 V | Hori zont al | 1-4 | 0-4 | Yes | 5s-12 | 30mi | 0.14 9 % | 82% | Bact eria ≥99. 999 % F | No |
| | | | | | | | No | | | | 85% | | No |
| | | | | | ≥4 | ≥4 | Yes | 5s-12 | 30mi | | 73% | | No |
| | | | | | | | No | | | | 78% | | No |
| | | | | Verti cal place | 1-4 | 0-4 | Yes | 5s-12 | 30mi | | 87% | | No |
| | | | | | | | No | | | | 90% | | No |
| | | | | | ≥4 | ≥4 | Yes | 5s-12 | 30mi | | 82% | | No |
| | | | | | | | No | | | | 84% | | No |
| 11 | 11 | >0. 49 V | 5.0 V | Hori zont al | 1-4 | 0-4 | Yes | 5s-12 | 30ini | 0.15 5%. | 81% | Bact eria ≥99. 999 % Fung | No |
| | | | | | | | No | | | | 86% | | No |
| | | | | | ≥4 | ≥4 | Yes | 5s-12 | 30mi | | 75% | | No |
| | | | | | | | No | | | | 8.0% | | No |
| | | | | Verti cal place | 1-4 | 0-4 | Yes | 5s-12 | 30mi | | 89% | | No |
| | | | | | | | No | | | | 91% | | No |
| | | | | | ≥4 | ≥4 | Yes | 5s-12 | 30mi | | 83% | | No |
| | | | | | | | No | | | | 83% | | No |
| 12 | 12 | >0. 49 V | 5.0 V | Hori zont al | 1-4 | 0-4 | Yes | 5s-12 | 30mi | 0.16 3 % | 87% | Bact eria ≥99. 999 % Fung | No |
| | | | | | | | No | | | | 90% | | No |
| | | | | | ≥4 | ≥4 | Yes | 5s-12 | 3Um | | 84% | | No |
| | | | | | | | No | | | | 87% | | No |
| | | | | Verti cal place | 1-4 | 0-4 | Yes | 5s-12 | 30mi | | 83% | | No |
| | | | | | | | No | | | | 90% | | No |
| | | | | | ≥4 | ≥4 | Yes | 5s-12 | 30mi | | 84% | | No |
| | | | | | | | No | | | | 85% | | No |

Under normal circumstances, protein removal care is performed on contact lenses once a week. By adjusting and coordinating various parameters, achieving no fading after 50 care cycles can meet the requirements for annual replacement contact lens care. The table also shows that as the initial pH value of the sodium chloride solution increases, its impact on fading decreases, and the protein removal efficiency improves.

Additionally, under experimental conditions using a physiological saline with a mass concentration of 0.9%, pH value greater than 8, starting voltage of 1.1V, contact lenses placed vertically in the care chamber, distance from the liquid surface greater than 4mm, distance from the anode greater than 4mm, and without electrode switching, working for 10 minutes, the effects of different initial physiological saline pH values and different stable voltages on ClO⁻ concentration were detected, as shown in Table 2 below:

**Table 2 Effects of different initial physiological saline pH values and different stable voltages on ClO⁻ concentration**

| Stable voltage | PH=8 /C_{ClO- WT}% | PH=9 /C_{ClO- WT}% | PH=10 /C_{ClO- WT}% | PH=11 /C_{ClO- WT}% | PH=12 /C_{ClO- WT}% |
|---|---|---|---|---|---|
| 3v | 0.0186 | 0.0193 | 0.0196 | 0.0231 | 0.0254 |
| 5v | 0.0665 | 0.0683 | 0.0702 | 0.0732 | 0.0749 |
| 6v | 0.0953 | 0.0973 | 0.0985 | 0.1086 | 0.1126 |
| 7v | 0.1375 | 0.1393 | 0.1432 | 0.1483 | 0.1537 |
| 8v | 0.1687 | 0.1712 | 0.1753 | 0J788 | 0.1807 |
| 9v | 0.1995 | 0.2 | 0.2021 | 0.2095 | 0.21 |
| 10v | 0.2247 | 0.2289 | 0.2326 | 0.2383 | 0.2413 |

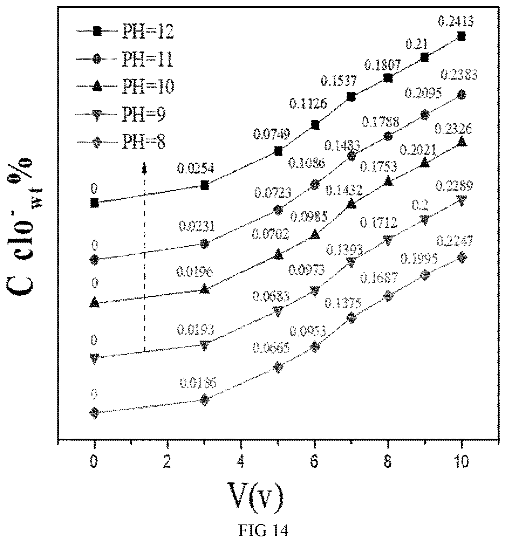

As can be seen from Table 2 and FIG. 14, the higher the initial pH value of the physiological saline, the higher the concentration of ClO⁻ in the solution. ClO⁻ aids in the sterilization and protein removal of contact lenses, meaning a higher pH value is more conducive to sterilization and protein removal of contact lenses. At the same time, a higher pH value helps reduce the impact on the color of contact lenses.

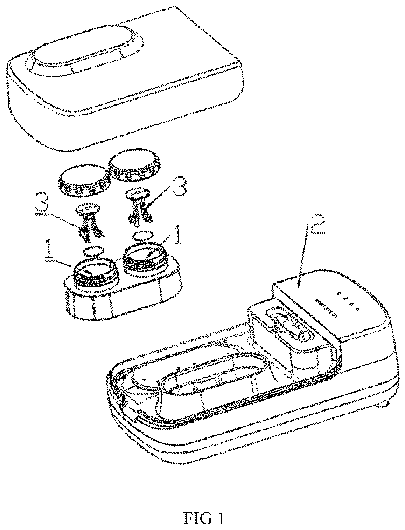

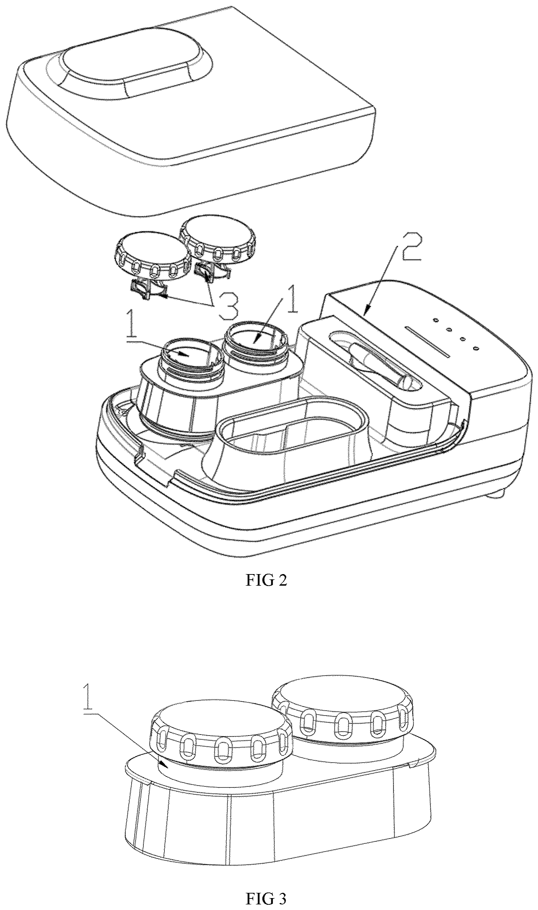

On the other hand, the present invention provides an electrochemical sterilization and protein removal device, as shown in FIG. 1-8, which includes a care chamber 1, a base 2, and a control system.

The care chamber 1 includes an accommodation slot 11, a sunken slot 12, a first electrode 13, and a second electrode 14. The sunken slot 12 is connected to the accommodation slot 11 and located below it. The first electrode 13 and second electrode 14 are both arranged within the accommodation slot 11. The base 2 can supply electrical energy to the first electrode 13 and second electrode 14.

When the first electrode and second electrode are energized, they have opposite polarities, with one being the cathode and the other the anode. Under working conditions where a sodium chloride solution with a pH value greater than 8 is added to the care chamber, an oxidation reaction occurs at the anode end, and a reduction reaction occurs at the cathode end. The cathode electrolyzes H₂O in the sodium chloride solution to produce H₂ and OH⁻. The anode electrolyzes Cl⁻ in the sodium chloride solution to produce Cl₂, which dissolves in the solution to form HCl and HClO. The ClO⁻ can sterilize contact lenses and degrade denatured proteins and some non-denatured proteins on the lenses. The degraded denatured proteins and some non-denatured proteins move toward the electrode with the opposite charge under the electric field force formed between the anode and cathode. Non-degraded non-denatured proteins also move toward the electrode with the opposite charge under the electric field force, achieving sterilization and protein removal of the contact lenses. It should be noted that proteins on contact lenses include denatured and non-denatured proteins. When contact lenses are placed in the solution, non-denatured proteins generally detach from the lenses and enter the solution, where they may or may not be degraded. Non-denatured proteins, whether degraded or not, move toward the electrode with the opposite charge under the electric field force. Denatured proteins adhere to the contact lenses. Degraded denatured proteins detach from the lenses and enter the solution, then move toward the electrode with the opposite charge under the electric field force.

The control system can control the operation of the device.

Preferably, the NaCl solution is a physiological saline with a mass concentration of 0.9%. The starting voltage for the first and second electrodes when connected to the power supply is not less than 1.1V, the stable voltage range after connection is 3.5-6.5V, and the working time after connection is 10-60 minutes. During operation, the mass concentration of ClO⁻ in the solution within the care chamber is maintained at 0.1%-0.4% to achieve optimal sterilization and protein removal effects and the safest operating environment.

Pour the sodium chloride solution into the care chamber. After connecting the first electrode and the second electrode to the power supply, the following reactions occur at the cathode and anode:

Cathode: 2H₂O + e = H2↑ + 2OH⁻(I)

Anode: 2Cl⁻ - e → Cl₂↑ (II)

Cl₂+H₂O = HCl+HClO (III)

The resulting HClO undergoes the following reaction:

2H ClO = 2HCL+O₂↑ (IV)

Na++ClO⁻= NaClO (V)

NaClO+H₂O = HClO+NaOH (VI)

When the sodium chloride solution is in a neutral environment, the solution mainly contains a mixture of NaClO, NaOH, and HClO. When the sodium chloride solution is in an alkaline environment, the solution primarily consists of NaClO and NaOH. In an alkaline environment, the dynamic equilibrium of the reversible reaction in Equation VI shifts to the left, resulting in a higher concentration of NaClO and a lower concentration of HClO. NaClO exhibits milder sterilization and protein removal effects without bleaching properties, preventing lens discoloration. The ClO⁻ ions in NaClO can sterilize and degrade proteins, and, in combination with electrophoresis, achieve protein removal effects. HClO has a sterilizing effect but also a bleaching effect. A high concentration of HClO can cause lens discoloration, while a lower concentration of HClO significantly reduces its impact on the color of contact lenses.

In a preferred embodiment, the first electrode and the second electrode are symmetrically arranged in the accommodation slot 11, both positioned close to the sidewall of the accommodation slot 11 to achieve better protein removal effects. In the example, both the first electrode and the second electrode are conductive probes. To facilitate assembly and sealing, the conductive probes are designed as split-type components. It should be noted that the electrodes may also be conductors of other shapes.

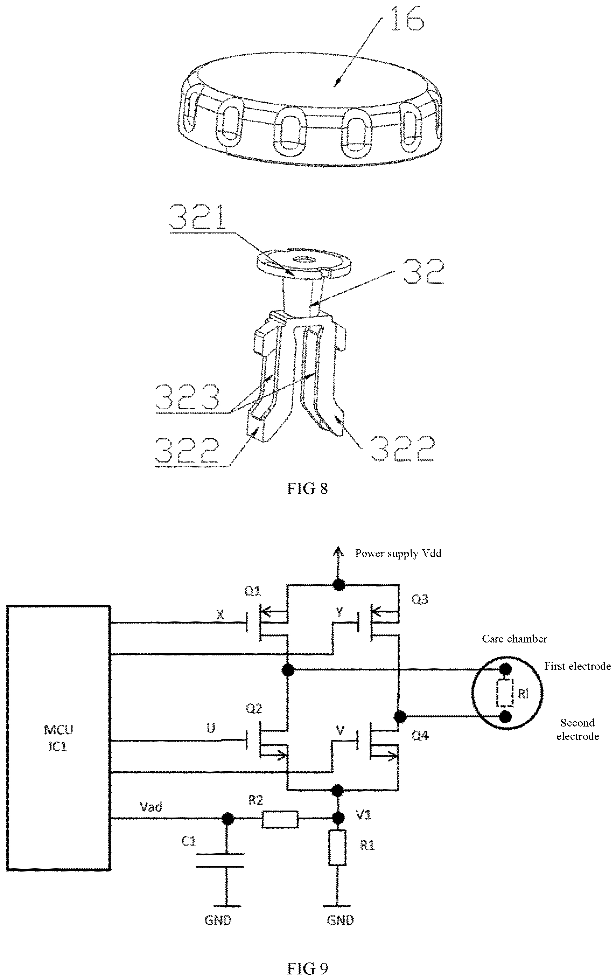

In a preferred embodiment, the control system is provided with an electrode switching module. After the first electrode and the second electrode are energized, one serves as the cathode and the other as the anode. The electrode switching module can switch the first electrode and the second electrode between the cathode and the anode through a control circuit. Preferably, the first electrode and the second electrode switch between the cathode and the anode every 0.5 to 2 minutes. FIG. 9 shows a schematic diagram of the electrode operation and switching circuit, and Table 3 below presents the driving logic diagram of the circuit:

**Table 3 Circuit Logic Driving Table for the First Electrode and the Second Electrode**

| Status of the First Electrode and the Second Electrode | | First Status | Second Status | Third Status |
|---|---|---|---|---|
| Drive Upper Arm | X | H | L | L |
| | Y | L | H | L |
| Driven lower arm | U | L | H | L |
| | V | H | L | L |
| Care chamber | First electrode | + | - | Floating |
| | Second electrode | - | Ten | Floating |

In the circuit schematic diagram of FIG. 9, IC1 is the microcontroller unit (MCU), Q1 and Q3 are both N-channel MOSFETs, Q2 and Q4 are both P-channel MOSFETs, R1 and R2 are resistors, Cl is a capacitor, the care chamber contains physiological saline with a mass concentration of 0.9%, the contact lens is placed in the care chamber, and there is a simulated electrode R' between the first electrode and the second electrode. The level signals driven by the MCU: H is high level, L is low level.

As shown in the circuit logic driving table of Table 3, the first state: the MCU's driving upper arm X is high level (H), driving upper arm Y is low level (L), the MCU's driving lower arm U is low level (L), driving upper arm V is high level (H), then the current forms a current loop from power supply Vdd→Q1→R'→Q4→R1, at this time the first electrode of the care chamber is the positive pole (+), the second electrode is the negative pole (-), the current flows through R1, generating a divided voltage VI, which is filtered through R2 and C1, and enters the MCU AD sampling voltage Vad, the voltage at this port is used to determine whether it is a current loop and whether a current loop has been established. If Vad has voltage, the care chamber operates normally; if Vad voltage is 0V, the care chamber operates abnormally.

Second state: The upper arm X drive of the MCU is at low level (L), the upper arm Y drive is at high level (H), the lower arm U drive of the MCU is at high level (H), and the upper arm V drive is at low level (L). Then, the current forms a loop from power supply Vdd→Q3→R'→Q2→R1. At this time, the first electrode of the care chamber is the negative pole (-), and the second electrode is the positive pole (+). The current flows through R1, generating a divided voltage VI, which is filtered through R2 and C1 and then enters the MCU AD sampling voltage Vad. The voltage at this port is used to determine whether a current loop is formed and whether a current loop is established. If Vad has voltage, the care chamber operates normally; if Vad voltage is 0V, the care chamber operates abnormally.

Third state: The upper arm X drive of the MCU is at low level (L), the upper arm Y drive is at low level (L), the lower arm U drive of the MCU is at low level (L), and the upper arm V drive is at low level (L). Then, no current loop is formed, and both the first electrode and the second electrode are in a floating state and uncharged, so the care chamber does not operate.

If the device operates continuously in the first state or the second state, the first electrode and the second electrode do not perform electrode switching. If the electrode switching module control circuit switches between the first state and the second state, the device operates in the electrode switching mode, and the first electrode and the second electrode switch between the cathode and the anode. The electrode switching of the first electrode and the second electrode is achieved through the above method.

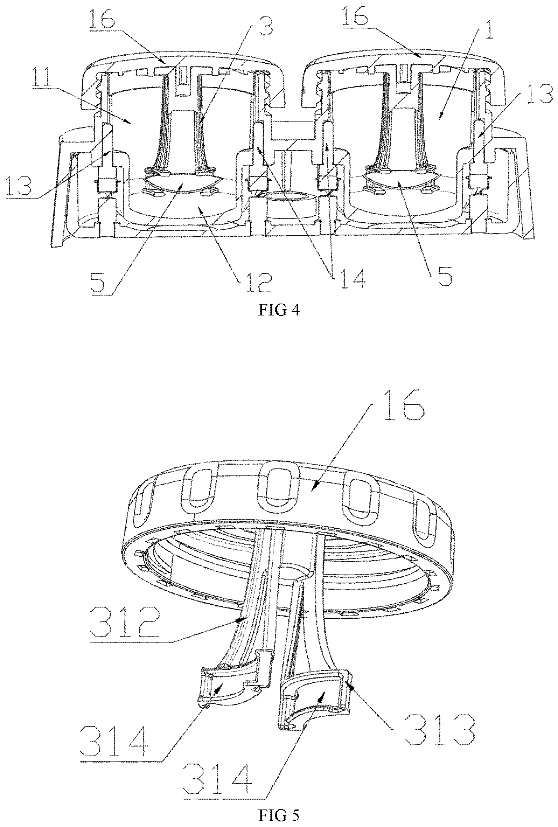

In a preferred embodiment, as shown in FIG. 4-8, it also includes a lens clip 3, which can secure the contact lens 5 in a chloride ion-containing solution.

In one example, the lens clip is a lateral lens clamp 31, which can secure the contact lens horizontally in a sunken slot 12 to prevent the contact lens from being too close to the electrode and oxidizing or fading. The height from the top of the contact lens to the liquid surface of the sodium chloride solution is not less than 4mm. The contact lens and the electrode are at different horizontal gradients, increasing the contact distance between the contact lens and the anode.

As shown in FIG. 4-6, the lateral lens clamp includes a mounting base 311, a support member 312, and a limit member 313. The support member 312 is vertically disposed on the mounting base 311. The limit member 313 is provided with a limit hole 314, and there are at least two limit members 313. The contact lens is positioned within the limit hole 314. During use, the length direction of the limit hole 314 is aligned with the horizontal direction. The limit hole serves to position the contact lens, ensuring that during the protein removal and sterilization process, the contact lens can be horizontally accommodated in the sodium chloride solution without movement or shaking, which could affect the protein removal and sterilization efficacy or damage the contact lens.

Preferably, the support member 312 is a flexible support body, facilitating the accommodation of the contact lens; the limit member 313 is a flexible limit member, preventing damage to the contact lens. In the example, the lateral lens clamp is a one-piece designed plastic lens clamp.

In one example, the lens clamp is a vertical lens clamp 32, which can position the contact lens vertically within the care chamber. The plane formed by the first electrode and the second electrode is perpendicular to the contact lens. The distance between the contact lens and the cathode probe or anode probe is not less than 4 mm, and the height from the top of the contact lens to the liquid surface of the sodium chloride solution is not less than 4 mm.

The purpose of maintaining a height of not less than 4 mm from the top of the contact lens to the liquid surface of the sodium chloride solution: during sterilization and protein removal of the contact lens, the anode can electrolyze Cl⁻ in the sodium chloride solution into Cl₂. Cl₂ rises from near the electrode and escapes from the liquid surface. After escaping, Cl₂ dissolves in the solution due to its own gravity and water solubility, producing HCl and HClO. Therefore, the concentration of HClO and Cl₂ is higher near the top of the liquid surface. When HClO accumulates to a certain concentration, it may affect the color of the lens. Thus, maintaining sufficient distance between the top of the contact lens and the liquid surface can reduce the rate of discoloration of the contact lens.

The purpose of maintaining a distance of not less than 4 mm between the contact lens and the cathode probe or anode probe: during sterilization and protein removal of the contact lens, the areas near the anode and cathode are in two different pH environments. Near the cathode, H₂O in the sodium chloride solution is electrolyzed to produce H₂ and OH⁻, e+2H₂O= H₂ and OH⁻. Near the anode, Cl⁻ in the sodium chloride solution is electrolyzed into Cl₂, Cl₂ dissolves in water to produce HCl and HClO, with a pH much less than 7, creating an acidic environment. Therefore, it is necessary to maintain sufficient distance between the contact lens and the anode; otherwise, the lens color may fade easily.

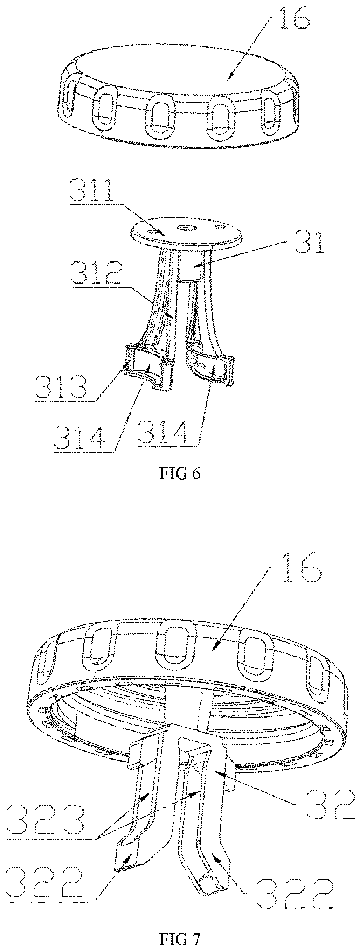

As shown in FIG. 7-8, the vertical lens clip includes a fixed base 321 and at least two accommodation members 322. The accommodation members 322 are vertically arranged on the fixed base 321, and the accommodation members 322 are provided with accommodation holes 323. During use, the length direction of the accommodation holes 323 is consistent with the vertical direction. There are at least two accommodation members 322, and the contact lens is confined within the accommodation holes 323 of the accommodation members 322. The accommodation holes serve to position the contact lens, ensuring that the contact lens can be vertically accommodated in the chloride ion-containing solution during the protein removal and sterilization process, without moving or shaking, which could affect the protein removal and sterilization efficacy or damage the contact lens. Preferably, the vertical lens clip adopts an eccentric design, so that when the contact lens is placed vertically, it is biased towards the cathode, keeping the contact lens away from Cl₂ and HClO, with the contacted liquid primarily being NaClO, thereby reducing the impact on the lens color and achieving relatively better sterilization and protein removal effects.

Preferably, the accommodation members 322 are flexible supports, which not only facilitate the accommodation of the contact lens but also avoid causing damage to the contact lens. In the example, the vertical lens clip is a one-piece designed plastic lens clamp.

Preferably, the care chamber is further provided with a cover body 16, which is closed on the care chamber 1. The lens clip 3 is fixedly installed on the cover body 16. In the example, the lens clip 3 is detachably installed on the cover body 16. When the contact lens is placed in the care chamber for sterilization and protein removal, the cover body 16 is closed on the care chamber 1. Further preferably, there are two care chambers 1, allowing simultaneous cleaning and care for the contact lenses of both eyes.

In the example, the base is provided with a PCB board, an energy storage component, and a control panel. The energy storage component can provide electrical energy to the device. The first electrode and the second electrode of the care chamber extend from the bottom of the care chamber to the outside of the care chamber. The base is provided with conductive contacts and a placement groove. The first electrode and the second electrode are respectively matched with corresponding conductive contacts. The conductive contacts and the control panel are electrically connected to the PCB board. The control panel can control the sterilization and protein removal work of the care chamber through the PCB board and the control system. During cleaning use, the care chamber is placed in the placement groove. Preferably, the care chamber is magnetically attracted to the placement groove.

In the example, the base is also provided with a rinsing chamber. After the sterilization and protein removal of the contact lens in the care chamber is completed, sterile physiological saline or multi-functional eye-safe care solution is poured into the rinsing chamber. The cover body is taken, and the contact lens fixed on the lens clip is transferred to the rinsing chamber. The rinsing chamber is then activated to clean the contact lens.

All features of the above components can be freely combined without conflict. In addition, structural changes, modifications, and variations of the components are also within the scope of protection of this patent.

The usage effects of this device are tested, including protein removal efficiency, bacterial sterilization rate, fungal sterilization rate, and impact tests on coatings.

### 1. Protein Removal Efficiency Test

The present invention uses 0.9% physiological saline, i.e., with a pH value of 7, and sends the device to an authoritative testing center (Suzhou BioTOP Biotechnology Service Co., Ltd.) to test its protein removal efficiency. Under the experimental conditions of a starting voltage of 1.1V, a stable voltage of 5V, the contact lens placed vertically in the care chamber, the distance from the contact lens to the liquid surface greater than 4mm, the distance from the contact lens to the anode greater than 4mm, and no electrode switching, the device operates for 30 minutes. The report number is MR20220407-01, and the detected protein removal efficiency is greater than 90%.

### 2. Sterilization Rate

Includes bacterial sterilization efficiency test and fungal sterilization efficiency test. The experimental conditions are: using 0.9% physiological saline with a pH value of 7, a starting voltage of 1.1V, a stable voltage of 5V, the contact lens placed vertically in the care chamber, the distance from the contact lens to the liquid surface greater than 4mm, the distance from the contact lens to the anode greater than 4mm, and no electrode switching. For the detection of bactericidal efficiency, the device was sent to the authoritative testing center of Zhejiang Fangyuan Testing Group Co., Ltd. for testing its bactericidal efficiency against bacteria. The test report number is 2212421321. After 10 minutes of operation, the sterilization rates were measured as 99.999% for Escherichia coli 8099 and 99.999% for Staphylococcus aureus ATCC6538.

For the detection of fungicidal efficiency, the same device was sent to the authoritative testing center of Zhejiang Fangyuan Testing Group Co., Ltd. for testing its fungicidal efficiency. The test report number is 2212419201. After 5 minutes of operation, the sterilization rate for Candida albicans TCC10231 was measured as 99.999%; after 10 minutes of operation, the sterilization rate for Candida albicans T810231 was measured as 99.999%.

The longest testing period for the above test reports was up to 6 months. Due to time constraints, this invention demonstrates the test results with an initial pH value of 7 for the sodium chloride solution to substantiate that the sterilization and protein removal method using this device exhibits good protein removal rate and sterilization rate.

### 3. Impact on Contact Lens Coating

Using the technology of this device for sterilization and protein removal care of contact lenses, compared with market care solutions, there is also a significant difference in the impact on the contact lens coating. Under the experimental conditions of 0.9% physiological saline with an initial pH range of 8-12, starting voltage of 1.1V, stable voltage of 5V, contact lenses placed vertically in the care chamber with a distance greater than 4mm from the liquid surface and greater than 4mm from the anode, without electrode switching, and operating for 30 minutes, the contact lenses were cared for. After care, the impact of sterilization and protein removal on the contact lens coating was detected via scanning electron microscopy and elemental quantitative analysis. The comparative example involved soaking the contact lenses in AB solution for 30 minutes, and after care, the impact of sterilization and protein removal on the contact lens coating was similarly detected via scanning electron microscopy and elemental quantitative analysis. Moreover, the experimental and comparative examples involved multiple care tests to assess their impact.

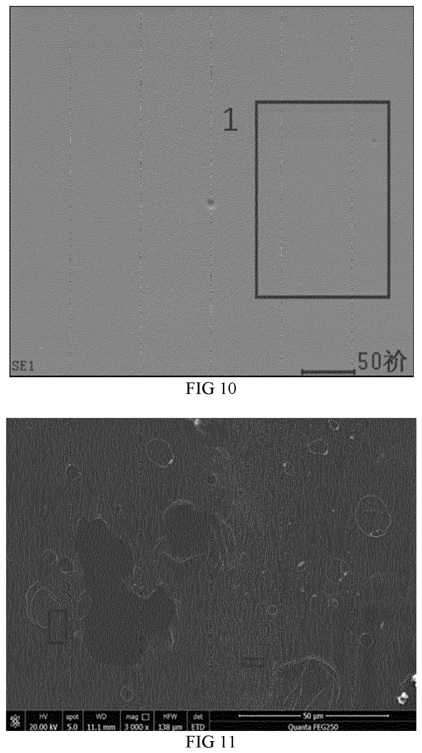

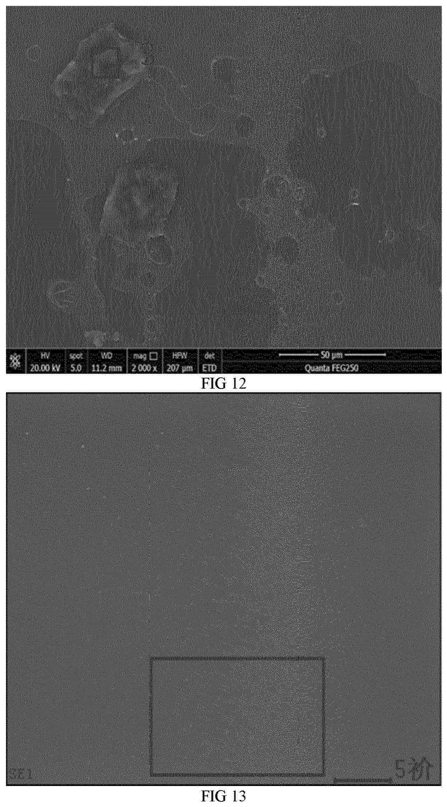

As shown in FIG. 10-13, FIG. 10 is the SEM image of a new contact lens, i.e., unworn and without sterilization and protein removal care; FIG. 11 is the SEM image of a contact lens after six months of care with AB solution; FIG. 12 is the SEM image of another contact lens after six months of care with AB solution; FIG. 13 is the SEM image of a contact lens after 300 cleanings with the electrochemical sterilization and protein removal device. Visually, the surfaces of new contact lenses that were unused and uncared for, as well as those cleaned and cared for with the electrochemical sterilization and protein removal device for nine months, appeared smoother, whereas the surfaces of contact lenses cared for with AB solution for six months appeared rougher. In this experiment, the time taken for 300 cleaning cycles using the electrochemical sterilization and protein removal device was 9 months.

The red box area in FIG. 10 is defined as Region 1, the two red box areas in FIG. 11 are defined as Region 2 and Region 3 respectively, and the red box area in FIG. 12 is defined as Region 4. The following are the elemental content analyses for Region 1, Region 1, Region 1, and Region 1 respectively.

**Table 4 Elemental analysis table of Region 1 on the surface of a new contact lens without use or care**

| **Element types in Region 1 of the new contact lens** | **Atomic percentage (At%)** | **Weight percentage (Wt%)** |
|---|---|---|
| C | 53.57% | 42.18% |
| N | 1.29% | 1.19% |
| O | 17.76% | 18.63% |
| F | 20.79% | 25.89% |
| Si | 6.58% | 12.11% |

**Table 5 Elemental analysis table of Region 2 and Region 3 on the surface of a contact lens after half a year of care with AB solution**

| **Element types** | **Atomic percentage (At%)** | | **Weight percentage (Wt%)** | |
|---|---|---|---|---|
| | **Region 2** | **Region 3** | **Region 2** | **Region 3** |
| C | 53.22% | 55.08% | 39.93% | 40.62% |
| 27.05%. | 27.83% | 0 | 6.50% | 0.00% |
| O | 9.99% | 11.06% | 9.99% | 10.86% |
| F | 13.93% | 17.73% | 16,53% | 20.69% |
| Si | 15.42% | 16.13% | 27.05% | 27.83% |

**Table 6 Elemental analysis table of another contact lens surface region 4 after six months of care with AB solution**

| **Element types in region 4** | **Atomic percentage (At%)** | **Weight percentage (Wt%)** |
|---|---|---|
| C | 63,66% | **54.53%** |
| N | 12.90% | **12.89%** |
| O | 15.94% | **18,18%** |
| F | 0.96% | 1.31% |
| Si | 6.53% | 13.09% |

**Table 7 Elemental analysis of contact lens surfaces after 300 times of care using electrochemical sterilization and protein removal device**

| **Element types** | **Atomic percentage (At%)** | **Weight percentage (Wt%)** |
|---|---|---|
| C | 54.37% | 42.07% |
| N | 1.90% | 1.72% |
| O | 13.35% | 13.76% |
| F | 21.34% | 26.12% |
| Si | 9.03% | 16.33% |

From the elemental content perspective, Area 2 of a contact lens after six months of care with AB solution and Area 1 of a new unused contact lens exhibit similar C content. Compared to Area 3 and Area 1, Area 2 and Area 1 show more similar total F and Si content, preliminarily indicating that Area 2 is the residual coating film on the contact lens surface, while Area 3 is the base material exposed after coating damage. The elemental content of Area 4 in another contact lens after six months of care with AB solution differs from Areas 1, 2, and 3, with a sharp decrease in F and Si content and an increase in N and O. The weight percentage increases dramatically, closely resembling the C, N, O ratio of proteins, reasonably suggesting the presence of significant protein content. The higher N content in Area 2 compared to Area 1 of the new contact lens may indicate protein adsorption during lens use. Thus, it can be inferred that after care with AB solution, the contact lens surface retains residual coating film and exposed base material, along with protein deposits. In contrast, contact lenses cared for 300 times with the electrochemical sterilization and protein removal device show surfaces as smooth as new lenses, with highly consistent atomic and weight percentages. This indicates no surface damage or protein deposits, making them nearly indistinguishable from new contact lenses.

In the description of this specification, references to terms such as "one embodiment," "some embodiments," "yet another embodiment," "another embodiment," "other embodiments," "example," "specific example," or "some examples" mean that the specific features, structures, materials, or characteristics described in connection with the embodiment or example are included in at least one embodiment or example of the present invention. In this specification, the schematic descriptions of the above terms do not necessarily refer to the same embodiment or example. Moreover, the specific features, structures, materials, or characteristics described may be combined in any one or more embodiments or examples in a suitable manner. Furthermore, those skilled in the art may combine and integrate the different embodiments or examples described in this specification.

Although the embodiments of the present invention have been shown and described above, it is to be understood that the above embodiments are exemplary and should not be construed as limiting the present invention. Those of ordinary skill in the art may make variations, modifications, and adaptations to the above embodiments within the scope of the present invention.

## Claims

1. An electrochemical sterilization and protein removal method, **characterized by** comprising the following steps:
S1: pouring a sodium chloride solution into a care chamber, wherein the care chamber is provided with at least a first electrode and a second electrode, both of which are chlorine evolution electrodes, the pH value of the sodium chloride solution ranges from 8 to 12, and a contact lens is placed in the sodium chloride solution within the care chamber;
S2: connecting the first electrode and the second electrode to a power supply, wherein one of the first electrode and the second electrode serves as an anode and the other as a cathode, an oxidation reaction occurs at the anode end, and a reduction reaction occurs at the cathode end, the cathode electrolyzes H₂O in the sodium chloride solution to produce H₂ and OH⁻; the anode electrolyzes Cl⁻ in the sodium chloride solution to produce Cl₂, and Cl₂ dissolves in the solution to produce HCl and HClO;
S3: controlling the pH value of the solution in the care chamber to range from 8 to 12, and controlling the mass concentration of Cl₂ in the solution to be 0.1%-0.4%, wherein ClO⁻can sterilize the contact lens and degrade denatured proteins on the contact lens, an electric field force is formed between the cathode and the anode, and the degraded denatured proteins move toward the electrode with the opposite charge under the action of the electric field force, thereby achieving sterilization and protein removal of the contact lens.

2. The electrochemical sterilization and protein removal method according to claim 1, **characterized in that** the NaCl solution is a physiological saline with a mass concentration of 0.9%;
in step S3, after the power supply is connected and the reaction proceeds for 10-60 minutes, the power supply is disconnected, and the contact lens care is completed.

3. The electrochemical sterilization and protein removal method according to claim 2, **characterized in that** the bottom heights of the first electrode and the second electrode are both higher than the bottom height of the care chamber, the contact lens is horizontally placed in the NaCl solution within the care chamber, the top height of the contact lens is lower than the bottom height of the probe, and the distance from the top of the contact lens to the liquid surface of the NaCl solution is not less than 4mm; or the contact lens is vertically placed in the NaCl solution within the care chamber, the distance from the contact lens to the anode is not less than 4mm, and the distance from the contact lens to the liquid surface of the NaCl solution is not less than 4mm.

4. The electrochemical sterilization and protein removal method according to claim 1, **characterized in that** in steps S2 and S3, after the first electrode and the second electrode are connected to the power supply, the first electrode and the second electrode switch between the cathode and the anode at certain time intervals.

5. The electrochemical sterilization and protein removal method according to claim 4, **characterized in that** the first electrode and the second electrode switch between the cathode and the anode every 0.5-2 minutes.

6. The electrochemical sterilization and protein removal method according to claim 1, **characterized in that** the NaCl solution further comprises an alkaline substance, the alkaline substance comprising one or more of NaOH, Na₂CO₃, and NaHCO₃, to adjust the pH value of the sodium chloride solution in step S1 to a range of 8-12.

7. The electrochemical sterilization and protein removal method according to claim 1, **characterized by** further comprising step S4;
S4: after the completion of step S3 care, rinsing the contact lens with sterile physiological saline or a multi-purpose eye care solution.

8. An electrochemical sterilization and protein removal device, **characterized by** comprising a care chamber (1) and a base (2);
wherein the care chamber (1) comprises an accommodation slot (11), a sunken slot (12), a first electrode, and a second electrode; the sunken slot (12) is connected to the accommodation slot (11), the sunken slot (12) is located below the accommodation slot (11), the first electrode and the second electrode are both disposed in the accommodation slot (11), and the base (2) is capable of supplying electrical energy to the first electrode and the second electrode;
when the first electrode and the second electrode are energized, the polarities of the first electrode and the second electrode are opposite, one being the cathode and the other being the anode, under the working condition of adding a sodium chloride solution with a pH value greater than 8 into the care chamber, an oxidation reaction occurs at the anode end, and a reduction reaction occurs at the cathode end, the cathode electrolyzes H₂O in the sodium chloride solution, electrolyze to produce H and OH⁻; the anode electrolyzes Cl₂ in the sodium chloride solution into Cl₂, and Cl₂ dissolves in the solution to produce HCl and HClO; the ClO⁻ can sterilize the contact lens and degrade denatured proteins on the contact lens, the degraded denatured proteins move toward the electrode with the opposite charge under the electric field force formed between the anode and the cathode to achieve sterilization and protein removal of the contact lens.

9. The electrochemical sterilization and protein removal device according to claim 8, **characterized in that** the first electrode and the second electrode are both chlorine evolution electrodes, and under working conditions, the mass concentration of ClO⁻ in the solution in the care chamber is 0.1%-0.4%;
the NaCl solution is physiological saline with a mass concentration of 0.9%, and the working time after power supply is connected is 10-60 minutes;
the first electrode and the second electrode are symmetrically arranged in the accommodation slot (11), and both the first electrode and the second electrode are close to the side wall of the accommodation slot (11).

10. The electrochemical sterilization and protein removal device according to claim 8, **characterized in that** it further comprises a lateral lens clamp (31), the lateral lens clamp (31) can limit the contact lens to be placed horizontally in the sunken slot (12) to prevent the contact lens from being too close to the electrode and oxidizing and fading, the distance between the top of the contact lens and the liquid surface of the sodium chloride solution is not less than 4mm; or further comprises a vertical lens clip (32), the vertical lens clip (32) can limit the contact lens to be placed vertically in the care chamber, the entire contact lens is arranged at a vertical angle to the first electrode and the second electrode, the distance between the contact lens and the anode is not less than 4mm, and the distance between the top of the contact lens and the liquid surface of the sodium chloride solution is not less than 4mm.

11. The electrochemical sterilization and protein removal device according to claim 10, **characterized in that** the lateral lens clamp (31) comprises a mounting base (311), a support member (312), and a limit member (313), the support member (312) is vertically arranged on the mounting base (311), the limit member (313) is provided with a limit hole (314), during use, the length direction of the limit hole (314) is consistent with the horizontal direction, the limit member (313) has at least two, and the contact lens is limited in the limit hole (314).

12. The electrochemical sterilization and protein removal device according to claim 8, **characterized in that** it further comprises a control system, the control system is provided with an electrode switching module, and the electrode switching module can switch the first electrode and the second electrode between the cathode and the anode through circuit control.
